# EUROPEAN PATENT APPLICATION

(11) **EP 4 817 537 A1**
(43) Date of publication of application: **07.10.2026**
(21) Application number: 25869707.7
(22) Date of filing: 01.07.2025
(51) Int. Cl.: A61B 17/34

(54) **CONTROL METHOD, CONTROL SYSTEM AND CONTROL APPARATUS FOR INSUFFLATOR, AND INSUFFLATOR**

(30) Priority: 19.02.2025 CN 202510179897
(71) Applicant: Microcure (Suzhou) Medical Technology Co., Ltd., Suzhou, Jiangsu 215000 (CN)
(72) Inventor: LIU, Yuxi, Suzhou, Jiangsu 215000 (CN); HE, Xusheng, Suzhou, Jiangsu 215000 (CN); ZHU, Mingshan, Suzhou, Jiangsu 215000 (CN)
(74) Representative: Vogelbruch, Keang
(86) International application number: PCT/CN2025/106522
(87) International publication number: WO 2026/174705

(57) **Abstract**

The present invention relates to the technical field of medical instruments, and provides a control method, control system, control apparatus for an insufflator, and an insufflator. The control method for an insufflator includes the following steps: acquiring a monitored pressure read by an insufflator; when the monitored pressure is greater than a first pressure threshold and a time duration for which it is maintained is greater than or equal to a first preset time duration, acquiring a pressure change rate within the first preset time duration; if the pressure change rate is greater than a first pressure change rate threshold, determining it as an expected fluctuation and initiating a first adjustment procedure; and if the pressure change rate is less than or equal to the first pressure change rate threshold, determining it as continuous change and initiating a second adjustment procedure. By using the pressure change rate to more carefully distinguish the cause of overpressure, different adjustment procedures may be adopted, allowing for targeted adjustment of expected fluctuating overpressure situations, thereby avoiding the problems of high wear of equipment components and long-time consumption for equipment pressure stabilization.

## Description

### CROSS REFERENCE TO RELATED APPLICATION

The present invention claims the priority to the Chinese patent application with the filing No. 2025101798977 filed with the China National Intellectual Property Administration on February 19, 2025, and entitled "CONTROL METHOD, CONTROL SYSTEM, CONTROL APPARATUS FOR AN INSUFFLATOR, AND INSUFFLATOR", which is incorporated herein by reference in entirety.

### TECHNICAL FIELD

The present invention relates to the technical field of medical instruments, and in particular, to a control method, control system, control apparatus for an insufflator, and an insufflator.

### BACKGROUND

In existing pneumoperitoneum equipment, to maintain stability of intra-abdominal pressure, a pressure monitoring and adjustment mechanism is normally employed. The mechanism has a specific pressure threshold. Once it is monitored that the intra-abdominal pressure fluctuates and exceeds this threshold, the equipment immediately initiates a deflation procedure for adjustment. However, in certain specific scenarios, for example, when a physician presses a patient's body during surgery, it may cause overpressure in the intra-abdominal pressure, and such fluctuation often exceeds the preset threshold. According to the traditional method for pneumoperitoneum stabilization, the equipment would rapidly deflate in this situation, and after the pressing action ends, a large amount of gas supplementation is needed to restore pressure balance within the pneumoperitoneum.

This traditional method for pneumoperitoneum stabilization has obvious disadvantages. On one hand, as long as the intra-abdominal pressure exceeds the threshold, the equipment inevitably performs deflation and gas supplementation actions, which undoubtedly increases the usage frequency of components, leads to higher component wear, and increases the maintenance cost and failure risk of the equipment. On the other hand, frequent deflation followed by gas supplementation prolongs the time required for the pneumoperitoneum to regain stability, thereby slowing down the overall progress of the surgery. Especially when the intra-abdominal pressure fluctuation caused by a physician's pressing is relatively brief, this problem of delayed stabilization becomes more prominent. After pressing, the physician often hopes the pneumoperitoneum can stabilize more quickly to better proceed with subsequent surgical operations.

Therefore, there is an urgent need for a new control method for an insufflator, which can effectively solve the problems of high equipment component wear and long-time consumption for stabilizing intra-abdominal pressure due to anticipated fluctuations, such as those caused by a physician's pressing, thereby improving surgical efficiency and reducing equipment wear.

### SUMMARY

An objective of the present invention is to provide a control method for an insufflator, so as to solve the technical problems existing in the prior art of high equipment component wear and long-time consumption for stabilizing equipment due to expected fluctuations.

In a first aspect, an embodiment of the present invention provides a control method for an insufflator, including the following steps: acquiring a monitored pressure read by an insufflator; when the monitored pressure is greater than a first pressure threshold and a time duration for which it is maintained is greater than or equal to a first preset time duration, acquiring a pressure change rate within the first preset time duration; if the pressure change rate is greater than a first pressure change rate threshold, determining it as expected fluctuation and initiating a first adjustment procedure; if the pressure change rate is less than or equal to the first pressure change rate threshold, determining it as continuous change and initiating a second adjustment procedure.

Further, the acquiring a monitored pressure read by an insufflator includes: acquiring sampling pressures and sampling time points corresponding to the sampling pressures; and taking an average value of sampling pressures at every N consecutive sampling time points as the monitored pressure, where N ≥ 1.

Further, the acquiring a pressure change rate within the first preset time duration includes: within the first preset time duration, including M values of the monitored pressures, dividing a difference between adjacent monitored pressures by an interval time between the adjacent monitored pressures to obtain M-1 pressure change rates; and taking a maximum value among the M-1 pressure change rates as the pressure change rate within the first preset time duration.

Further, the first adjustment procedure includes: when the monitored pressure is less than or equal to a second pressure threshold and a time duration for which it is maintained is greater than a second preset time duration, initiating a first pressure reduction procedure; and when the monitored pressure is greater than the second pressure threshold, initiating a second pressure reduction procedure.

Further, the first adjustment procedure further includes: when the monitored pressure is less than a second pressure threshold and a time duration for which it is maintained is less than a second preset time duration, issuing a first prompt signal; when the monitored pressure is less than or equal to the second pressure threshold and the time duration for which it is maintained is greater than the second preset time duration, issuing a second prompt signal; and when the monitored pressure is greater than the second pressure threshold, issuing a third prompt signal.

Further, the first pressure reduction procedure includes: opening a first pressure relief valve; the second pressure reduction procedure includes: opening the first pressure relief valve and a second pressure relief valve.

Further, the second adjustment procedure includes: when the monitored pressure is less than or equal to a second pressure threshold, initiating a third pressure reduction procedure and initiating a first counting procedure; when the monitored pressure is greater than the second pressure threshold, initiating a fourth pressure reduction procedure and initiating a second counting procedure.

Further, the second adjustment procedure further includes: when the monitored pressure is less than or equal to a second pressure threshold, issuing a fourth prompt signal; and when the monitored pressure is greater than the second pressure threshold, issuing a fifth prompt signal.

Further, the third pressure reduction procedure includes: opening a first pressure relief valve; the fourth pressure reduction procedure includes: opening the first pressure relief valve and a second pressure relief valve.

In a second aspect, an embodiment of the present invention further provides a control system for an insufflator, including: a pressure monitoring module, configured to acquire a monitored pressure read by an insufflator and determine whether the monitored pressure is greater than a first pressure threshold and a time duration for which it is maintained is greater than or equal to a first preset time duration; a pressure change rate calculation module, connected to the pressure monitoring module and configured to, when the pressure monitoring module determines that the monitored pressure is greater than the first pressure threshold and the time duration for which it is maintained is greater than or equal to the first preset time duration, acquire a pressure change rate within the first preset time duration; a determination module, connected to the pressure change rate calculation module and configured to determine whether it is expected fluctuation or continuous change according to the pressure change rate calculated by the pressure change rate calculation module; wherein the determination module is configured to determine it as expected fluctuation when the pressure change rate is greater than a first pressure change rate threshold, and determine it as continuous change when the pressure change rate is less than or equal to the first pressure change rate threshold; and an adjustment control module, connected to the determination module and configured to be initiated to execute a first adjustment procedure when the determination module determines it as expected fluctuation, and to be initiated to execute a second adjustment procedure when the determination module determines it as continuous change, so that the intra-abdominal pressure remains stable.

In a third aspect, an embodiment of the present invention further provides a control apparatus for an insufflator, including a processor, a memory, and a computer program stored in the memory and executable on the processor, wherein the computer program, when executed, implements the method steps in the control method for an insufflator described above.

In a fourth aspect, an embodiment of the present invention further provides an insufflator, including: the aforementioned control apparatus for an insufflator; a pressure sensor, configured to measure an intra-abdominal pressure; and a first pressure relief valve and a second pressure relief valve, configured to control intra-abdominal pressure relief; wherein the control apparatus for an insufflator is electrically connected to each of the pressure sensor, the first pressure relief valve, and the second pressure relief valve.

The embodiments of the present invention have at least the following technical effects.

The control method for pneumoperitoneum stability provided in the embodiments of the present invention can, during a surgical phase where the pneumoperitoneum needs to be maintained stable, restore the pneumoperitoneum to its originally pressure that needs to be maintained in the event of an intra-abdominal overpressure condition. First, it is necessary to acquire a monitored pressure of the pneumoperitoneum. A first pressure threshold and a first preset time duration are set in advance. When the monitored pressure is greater than the first pressure threshold and the time duration for which it is maintained is greater than or equal to the first preset time duration, a pressure change rate within the first preset time duration is acquired; if the pressure change rate is greater than a first pressure change rate threshold, it is determined as expected fluctuation and a first adjustment procedure is initiated; if the pressure change rate is less than or equal to the first pressure change rate threshold, it is determined as continuous change and a second adjustment procedure is initiated.

Since an expected fluctuant pressure increase caused by manual pressing has an obvious difference in the rate of pressure change from continuously changing overpressure caused by other reasons, it is necessary to preset a first pressure change rate threshold in advance. The causes for expected fluctuating overpressure and continuously changing overpressure are different. The cause of expected fluctuating overpressure is relatively clear, and the pneumoperitoneum needs to return to the expected pressure quickly after the expected fluctuation. The overpressure caused by continuous change may have various causes. One type is unpredictable changes in the patient themselves, such as decreased compliance of the patient's abdominal wall, edema or congestion of abdominal organs, etc. Another type is an insufflator performance issue. For example, if a gas delivery pipeline in the insufflator is bent or blocked, it may also cause the intra-abdominal pressure to rise slowly. By using the pressure change rate to more carefully distinguish the cause or situation of the overpressure, different adjustment procedures may be adopted. This can largely avoid the problem of high equipment component wear, and targeted adjustment can be made for the expected fluctuating overpressure situation, avoiding the problem of long-time consumption for the equipment to stabilize intra-abdominal pressure after expected fluctuation, thereby improving surgical efficiency and reducing equipment wear.

### BRIEF DESCRIPTION OF DRAWINGS

To explain the technical solutions in the embodiments of the present invention or the prior art more clearly, the drawings used in the description of the embodiments or the prior art will be briefly introduced below. Obviously, the drawings in the following description are some embodiments of the present invention. For those of ordinary skill in the art, other drawings may be obtained based on these drawings without creative effort.
FIG. 1 is a schematic flowchart of a control method for an insufflator provided by an embodiment of the present invention;
FIG. 2 is a schematic flowchart for acquiring a monitored pressure read by an insufflator according to an embodiment of the present invention;
FIG. 3 is a schematic flowchart for acquiring a pressure change rate within a first preset time duration provided by an embodiment of the present invention;
FIG. 4 is a schematic flowchart of a part of a first adjustment procedure provided by an embodiment of the present invention;
FIG. 5 is a schematic flowchart of another part of the first adjustment procedure provided by an embodiment of the present invention;
FIG. 6 is a schematic flowchart of a part of a second adjustment procedure provided by an embodiment of the present invention;
FIG. 7 is a schematic flowchart of another part of the second adjustment procedure provided by an embodiment of the present invention; and
FIG. 8 is a schematic structural diagram of a control system for an insufflator provided by an embodiment of the present invention.

### DETAILED DESCRIPTION OF EMBODIMENTS

The technical solutions of the present invention will be described clearly and completely below in combination with the embodiments. Apparently, the described embodiments are only part, not all of the embodiments of the present invention. Based on the embodiments of the present invention, all other embodiments obtained by a person of ordinary skill in the art without creative effort shall fall within the protection scope of the present invention.

Those skilled in the art can understand that, unless otherwise defined, all terms (including technical terms and scientific terms) used herein have the same meaning as commonly understood by those of ordinary skill in the art to which the present invention belongs. It should also be understood that terms such as those defined in general dictionaries should be understood to have meanings consistent with their meanings in the context of the prior art, and will not be interpreted in an idealized or overly formal sense unless specifically defined as herein.

Those skilled in the art can understand that the singular forms "a", "an", "said" and "the" used herein may also include plural forms unless specifically stated otherwise. It should be further understood that the term "include" used in the specification of the present invention indicates the presence of the stated features, integers, steps, operations, elements, and/or components, but does not preclude the presence or addition of one or more other features, integers, steps, operations, elements, components, and/or combinations thereof. The term "and/or" used herein includes all of the one or more associated listed items, or any and all combinations of the one or more associated listed items.

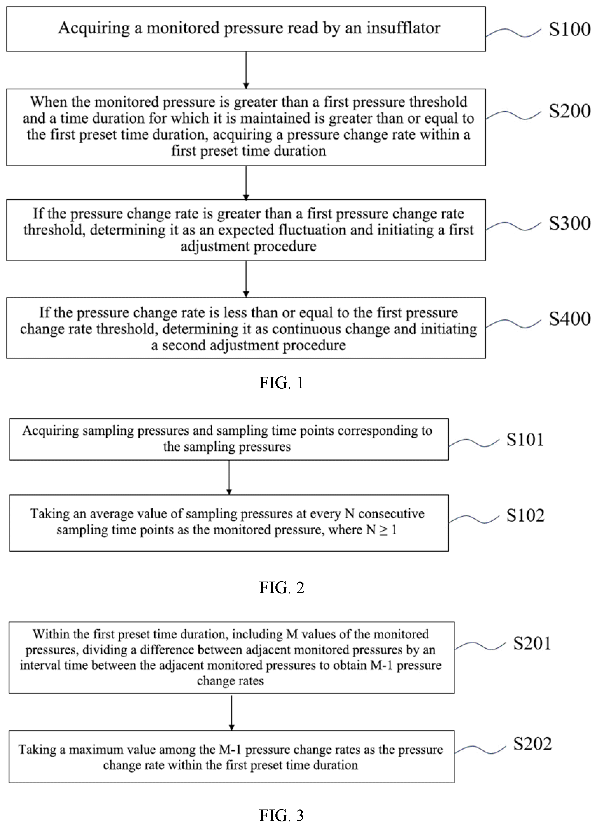

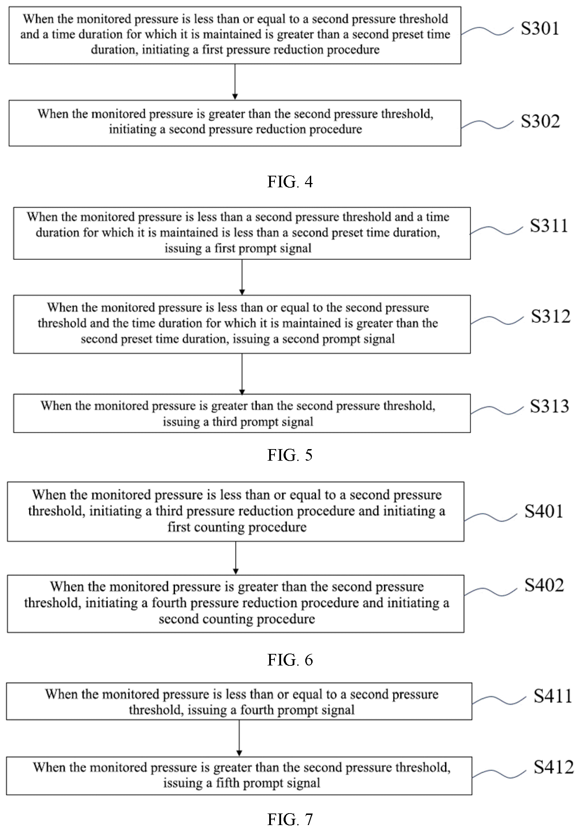

Referring to FIGS. 1 to 7, an embodiment of the present invention provides a control method for an insufflator, including the following steps.

S100: acquiring a monitored pressure read by an insufflator.

In this embodiment, this control method is applicable during a surgical phase where the pneumoperitoneum needs to be maintained stable. If intra-abdominal overpressure occurs, this control method is employed to restore the pneumoperitoneum to its original pressure that needs to be maintained. First, it is necessary to acquire the monitored pressure read by the insufflator, which is essentially the actual real-time pressure of the pneumoperitoneum.

S200: when the monitored pressure is greater than a first pressure threshold and a time duration for which it is maintained is greater than or equal to a first preset time duration, acquiring a pressure change rate within the first preset time duration.

Here, the first pressure threshold and the first preset time duration need to be set in advance. If the pressure that the pneumoperitoneum needs to be maintained, i.e., the expected pressure, is X mmHg, then the first pressure threshold may be set to (X+2) mmHg, and the first preset time duration may be set to 0.5 s. That is, if the difference between the monitored pressure of the pneumoperitoneum and the expected pressure of the pneumoperitoneum exceeds 2 mmHg, and this difference remains above 2 mmHg for 0.5 s, or when the monitored pressure of the pneumoperitoneum is greater than (X+2) mmHg and remains for 0.5 s, then at this moment, the pressure change rate within these 0.5 s should be calculated and obtained. Of course, setting the first pressure threshold to (X+2) mmHg and the first preset time duration to 0.5 s here is only an example for ease of understanding and is not a limitation on its value. Specifically, more appropriate values may be assigned based on the actual intra-abdominal pressure changes during surgery. This first pressure threshold is greater than the expected pressure but must be less than the maximum pressure that the human abdominal cavity can withstand.

S300: if the pressure change rate is greater than a first pressure change rate threshold, determining it as an expected fluctuation and initiating a first adjustment procedure.

S400: if the pressure change rate is less than or equal to the first pressure change rate threshold, determining it as continuous change and initiating a second adjustment procedure.

Since an expected fluctuation caused by manual pressing leads to a sudden intra-abdominal pressure increase, which has an obvious difference in the rate of pressure change from continuously changing overpressure caused by other reasons, it is necessary to preset a first pressure change rate threshold in advance. For example, the first pressure change rate threshold may be set to 2 mmHg/s. If the pressure change rate is greater than 2 mmHg/s, it is determined as expected fluctuation; if the pressure change rate is less than or equal to 2 mmHg/s, it is determined as continuous change. The causes for expected fluctuating overpressure and continuously changing overpressure are different. The cause of expected fluctuating overpressure is relatively clear, and the pneumoperitoneum needs to return to the expected pressure quickly after the expected fluctuation. The overpressure caused by continuous change may have various causes. One type is changes in the patient themselves, such as decreased compliance of the patient's abdominal wall (after pneumoperitoneum establishment, as the surgical operation proceeds, the abdominal wall gradually adapts to the intra-abdominal pressure, its elastic retraction force weakens, leading to a reduction in the relative space within the abdominal cavity, thus slowly increasing the intra-abdominal pressure), or unpredictable conditions such as edema or congestion of abdominal organs. Another type is an insufflator performance issue. For example, if a gas delivery pipeline in the insufflator is bent or blocked, it may also cause a slow rise in intra-abdominal pressure. By using the pressure change rate to more carefully distinguish the cause or situation of the overpressure, different adjustment procedures may be adopted. This can largely avoid the problem of high equipment component wear, and targeted adjustment can be made for the expected fluctuating overpressure situation, avoiding the problem of long-time consumption for the equipment to stabilize intra-abdominal pressure after expected fluctuation, thereby improving surgical efficiency and reducing equipment wear.

In this embodiment, step S100 specifically includes:
S101: acquiring sampling pressures and sampling time points corresponding to the sampling pressures; and
S102: taking an average value of sampling pressures at every N consecutive sampling time points as the monitored pressure, where N ≥ 1.

Generally, the sampling pressure of the equipment is very high-frequency, for example, pressure sampling is performed every 25ms. However, the monitored pressure of the pneumoperitoneum is to be displayed for the physician to observe. Taking the average value of sampling pressures at every N consecutive sampling time points as the monitored pressure, where N ≥ 1, and if N is 4, the monitored pressure of the pneumoperitoneum is the average value of sampling pressures within 100ms.

The advantage of this averaging approach is that, on one hand, it can reduce noise interference. Although high-frequency sampling can capture rapid pressure changes, it is also susceptible to various noises, such as electromagnetic interference in the surgical environment. These noises can cause instantaneous fluctuations in the sampled pressure values, making the monitored pressure data less smooth and stable. By taking the average value of N consecutive sampling pressure values, these instantaneous noise interferences can be effectively filtered out. When random noise exists, they partially cancel each other out across multiple sampling values, thereby making the final monitored pressure closer to the true intra-abdominal pressure level and providing more accurate monitoring results. On the other hand, it can provide a stable and reliable reference. During surgery, the physician needs to assess the safety and effectiveness of the surgical operation based on the intra-abdominal pressure. If the monitored pressure fluctuates frequently, it can make such assessments difficult for the physician and may even lead to misjudgment. The monitored pressure after averaging is more stable, which can provide a reliable reference basis for the physician and help them better control the surgical process. It can also assist the equipment in reducing unnecessary adjustments. The averaged monitored pressure can more accurately reflect the overall state of the pneumoperitoneum, making the adjustment of the insufflator more reasonable and necessary, and reducing unnecessary wear on the equipment.

In this embodiment, it is specifically explained that step S200 specifically includes:
S201: within the first preset time duration, including M values of the monitored pressures, dividing a difference between adjacent monitored pressures by an interval time between the adjacent monitored pressures to obtain M-1 pressure change rates; and
S202: taking a maximum value among the M-1 pressure change rates as the pressure change rate within the first preset time duration.

In this embodiment, taking the maximum value among the M-1 pressure change rates as the pressure change rate within the first preset time duration is mainly to ensure sensitivity to pressure changes and to determine the type of pressure change more accurately, where M ≥ 2. By taking the maximum value, it can be ensured that even rapid pressure changes occurring within a short period of time can be captured promptly. If it is an overpressure within the expected fluctuation, for example, the monitored pressure is greater than the first pressure threshold and lasts for the first preset time duration, but the monitored pressure does not keep increasing throughout the first preset time duration-it may become flat at a certain moment-taking the maximum value can avoid missing important information that might be overlooked by taking an average or a smaller value. During surgery, the maximum value can provide the most direct and obvious information about pressure changes, reducing misjudgments caused by inconspicuous pressure changes, and ensuring that the adjustment of the insufflator is more accurate and effective.

In this embodiment, it is specifically explained that step S300 specifically includes:
S301: when the monitored pressure is less than or equal to a second pressure threshold and a time duration for which it is maintained is greater than a second preset time duration, initiating a first pressure reduction procedure.

Optionally, the first pressure reduction procedure includes: opening a first pressure relief valve.

S302: when the monitored pressure is greater than the second pressure threshold, initiating a second pressure reduction procedure.

Optionally, the second pressure reduction procedure includes: opening the first pressure relief valve and a second pressure relief valve.

In this embodiment, a second pressure threshold and a second preset time duration need to be preset. The second pressure threshold is greater than the first pressure threshold, and the second preset time duration is greater than the first preset time duration. If the first pressure threshold is set to (X+2) mmHg, then the second pressure threshold may be set to (X+4) mmHg. The value of the second pressure threshold is more biased towards the maximum pressure that the pneumoperitoneum can bear but must be less than the maximum pressure value. The first preset time duration is for faster detection of changes, while the second preset time duration takes into consideration that if the fluctuation is caused by only a very brief press, overreaction of the equipment should be avoided. For example, the second preset time duration is set to 3 s. If the intra-abdominal overpressure is greater than the first pressure threshold but less than the second pressure threshold, and the duration is only 2 s, which is less than the second preset time duration, then the equipment does not need to initiate a pressure reduction procedure at this time. This is because such short-term, small-range overpressure will not cause significant harm to the human body, and the overpressure phenomenon will disappear after the press stops. Furthermore, because the equipment does not perform pressure reduction, the pneumoperitoneum will quickly restore its original pressure level, which also reduces overreaction of the equipment, reduces component wear, and frequent valve switching can also generate additional noise. This approach can also reduce noise generation.

When the monitored pressure is less than or equal to the second pressure threshold and the time duration for which it is maintained is greater than the second preset time duration, initiating the first pressure reduction procedure is to ensure that even if the range of overpressure is relatively small, if it lasts too long, it may still harm the human body, so pressure reduction is necessary at this time. When the monitored pressure is greater than the second pressure threshold, it indicates that the intra-abdominal pressure is closer to a critical value. In this case, pressure reduction is mandatory to prevent irreversible damage caused by excessively high intra-abdominal pressure. These two situations are different. Therefore, the adopted pressure reduction procedures are also different. The first pressure reduction procedure only opens the first pressure relief valve, while the second pressure reduction procedure needs to open both the first and the second pressure relief valves. The gas flow rate of the first pressure relief valve is less than that of the second pressure relief valve. Therefore, the pressure relief speed of the first pressure relief valve is slower than that of the second pressure relief valve. The first pressure reduction procedure can achieve pressure reduction without deflating too much gas, allowing the intra-abdominal pressure to stabilize more quickly after the press ends. The second pressure reduction procedure can ensure surgical safety to the greatest extent. Overall, they make the stabilization of pneumoperitoneum more efficient with minimal loss.

In this embodiment, it is specifically explained that step S300 further includes:
S311: when the monitored pressure is less than the second pressure threshold and the time duration for which it is maintained is less than the second preset time duration, issuing a first prompt signal;
S312: when the monitored pressure is less than or equal to the second pressure threshold and the time duration for which it is maintained is greater than the second preset time duration, issuing a second prompt signal; and
S313: when the monitored pressure is greater than the second pressure threshold, issuing a third prompt signal.

In this embodiment, it is considered that if the physician only observes the monitored pressure during surgery, they cannot intuitively grasp the dynamic status of the equipment, which could also pose a potential risk during surgery. Therefore, prompt signals need to be added. The prompt signals may appear in the form of sound, light, text, or even a combination thereof. The first, second, and third prompt signals are all different. The following provides textual examples, the first, second, and third prompt signals may be: "Expected Fluctuation in Progress", "Expected Fluctuation Time-out", and "Expected Fluctuation Overpressure", respectively. The prompt "Expected Fluctuation in Progress" indicates that the equipment has detected a pressure fluctuation caused by, for example, manual pressing, but will not deflate; it is for prompting purposes only. The prompt "Expected Fluctuation Time-out" can alert the physician and indicates that the equipment will open the first pressure relief valve to reduce pressure, and close it when the monitored pressure falls below the first pressure threshold. The prompt "Expected Fluctuation Overpressure" can alert the physician and indicates that the equipment will open both the first and second pressure relief valves to reduce pressure, and close the first and second pressure relief valves when the monitored pressure falls below the first pressure threshold.

In this embodiment, it is specifically explained that step S400 specifically includes:
S401: when the monitored pressure is less than or equal to the second pressure threshold, initiating a third pressure reduction procedure and initiating a first counting procedure.

Optionally, the third pressure reduction procedure includes: opening a first pressure relief valve.

Optionally, the first counting procedure includes: if the number of times the third pressure reduction procedure is initiated exceeds a first preset number of times within a third preset time duration, issuing a first warning signal.

S402: when the monitored pressure is greater than the second pressure threshold, initiating a fourth pressure reduction procedure and initiating a second counting procedure.

Optionally, the fourth pressure reduction procedure includes: opening the first pressure relief valve and a second pressure relief valve.

Optionally, the second counting procedure includes: if the number of times the fourth pressure reduction procedure is initiated exceeds a second preset number of times within a fourth preset time duration, issuing a second warning signal.

In this embodiment, since the cause of continuous changing pressure increase is unclear, using the comparison between the monitored pressure and the second pressure threshold as the criterion for determining which pressure reduction procedure to execute provides a more reliable safeguard, with the third and fourth pressure reduction procedures being distinct from each other. At the same time, because the cause of the pressure increase is unclear but it continues to increase, besides pressure relief, it is also necessary to consider whether the continuous pressure increase is due to the patient's own condition or equipment-related factors.

Therefore, besides opening the first pressure relief valve when the monitored pressure is less than or equal to the second pressure threshold and closing the first pressure relief valve when the monitored pressure falls below the first pressure threshold, and opening both the first and second pressure relief valves when the monitored pressure is greater than the second pressure threshold and closing the first and second pressure relief valves when the monitored pressure falls below the first pressure threshold, it is also necessary to consider initiating a counting procedure. Because pressure changes caused by the patient's own condition are limited, if within a certain defined time period that follows, the number of times the same continuous change exceeds a set number of times-for example, if the third pressure reduction procedure is initiated five times within the next 10 minutes of surgery-it indicates that there is a probability that some equipment problem has occurred at this time, and a warning signal is issued to remind the physician to eliminate the potential risk in time. Here, the third preset time duration of 10 minutes and the first preset number of 5 times are only examples for ease of understanding and are not limitations on their assignment. The third and fourth preset time durations may be the same or different, the first and second preset numbers may be the same or different, and reasonable values may be set according to the actual surgical procedure.

In this embodiment, it is specifically explained that, step S400 further includes:
S411: when the monitored pressure is less than or equal to the second pressure threshold, issuing a fourth prompt signal; and
S412: when the monitored pressure is greater than the second pressure threshold, issuing a fifth prompt signal.

In this embodiment, the fourth and fifth prompt signals are distinct from each other, mainly to ensure that the physician can more intuitively grasp the dynamic status of the equipment during surgery. The prompt signals may also appear in the form of sound, light, text, or images, or even a combination thereof.

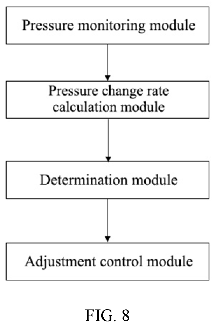

Referring to FIG. 8, an embodiment of the present invention provides a control system for an insufflator stabilization, including: a pressure monitoring module, configured to acquire a monitored pressure read by an insufflator and determine whether the monitored pressure is greater than a first pressure threshold and a time duration for which it is maintained is greater than or equal to a first preset time duration; a pressure change rate calculation module, connected to the pressure monitoring module and configured to, when the pressure monitoring module determines that the monitored pressure is greater than the first pressure threshold and the time duration for which it is maintained is greater than or equal to the first preset time duration, acquire a pressure change rate within the first preset time duration; a determination module, connected to the pressure change rate calculation module and configured to determine whether it is expected fluctuation or continuous change according to the pressure change rate calculated by the pressure change rate calculation module; wherein the determination module is configured to determine it as expected fluctuation when the pressure change rate is greater than a first pressure change rate threshold, and determine it as continuous change when the pressure change rate is less than or equal to the first pressure change rate threshold; and an adjustment control module, connected to the determination module and configured to be initiated to execute a first adjustment procedure when the determination module determines it as expected fluctuation, and to be initiated to execute a second adjustment procedure when the determination module determines it as continuous change, so that the intra-abdominal pressure remains stable.

In this embodiment, by using the pressure monitoring module, the pressure inside the pneumoperitoneum is monitored in real time, and it can be determined whether the monitored pressure is greater than the first pressure threshold and the time duration for which it is maintained is greater than or equal to the first preset time duration. Based on the determination result of the pressure monitoring module, then the pressure change rate within the first preset time duration is calculated by the pressure change rate calculation module. The determination module determines whether it is expected fluctuation or continuous change according to the pressure change rate calculated by the pressure change rate calculation module. The adjustment control module selects different adjustment procedures based on different overpressure causes to keep the intra-abdominal pressure stable. By using the pressure change rate to more carefully distinguish the cause or situation of the overpressure, different adjustment procedures may be adopted. This can largely avoid the problem of high equipment component wear, and targeted adjustment can be made for the expected fluctuating overpressure situation, avoiding the problem of long-time consumption for the equipment to stabilize intra-abdominal pressure after expected fluctuation, thereby improving surgical efficiency and reducing equipment wear.

The functions of each module in the control system for an insufflator described in this embodiment are the same as the method steps of the aforementioned control method for an insufflator. Therefore, details not described in this embodiment can be found in the specific description of the aforementioned control method for an insufflator and FIGS. 1 to 7, and are not repeated here.

An embodiment of the present invention further provides a control apparatus for an insufflator, including a processor, a memory, and a computer program stored in the memory and executable on the processor, wherein the computer program, when executed, implements the method steps in the aforementioned control method for an insufflator.

By means of the computer program stored in the memory and executed on the processor, the cause or situation of overpressure is distinguished in more detail based on the pressure change rate, allowing different adjustment procedures to be adopted. This avoids the problem of high equipment component wear and the problem of long-time consumption for stabilizing intra-abdominal pressure after expected fluctuation, thereby improving surgical efficiency and reducing equipment wear.

The method steps implemented when the computer program in the control apparatus for an insufflator of this embodiment to be executed correspond to the steps of the aforementioned control method for an insufflator. Details not described in this embodiment can be found in the specific description of the aforementioned control method for an insufflator and FIGS. 1 to 7, and are not repeated here.

An embodiment of the present invention further provides an insufflator, including: the aforementioned control apparatus for an insufflator; a pressure sensor, configured to measure an intra-abdominal pressure; and a first pressure relief valve and a second pressure relief valve, configured to control intra-abdominal pressure relief; wherein the control apparatus for an insufflator is electrically connected to each of the pressure sensor, the first pressure relief valve, and the second pressure relief valve.

In this embodiment, the control apparatus for an insufflator inside the insufflator relies on the pressure sensor to monitor and acquire the intra-abdominal pressure in real time, thereby making determinations and controlling the first and second pressure relief valves, so that the intra-abdominal pressure remains stable while also avoiding the problems of high equipment component wear and long-time consumption for stabilizing intra-abdominal pressure after expected fluctuation.

Optionally, the insufflator further includes a reminder structure, which is configured to issue the first to fifth prompt signals and the first and second warning signals. The reminder structure is controlled by the control apparatus for an insufflator, facilitating the physician to better grasp the dynamic status of the equipment and eliminate potential safety hazards in time. The prompt structure may emit any one or a combination of sound, light, text, or images.

Similarly, details not described in this embodiment can be found in the specific description of the aforementioned control method for an insufflator, control system, control apparatus, and FIGS. 1 to 8, and are not repeated here.

Those skilled in the art can understand that the steps, measures, and schemes in the various operations, methods, and processes discussed in the present invention may be alternated, modified, combined, or deleted. Further, other steps, measures, and schemes in the various operations, methods, and processes already discussed in the present invention may also be alternated, modified, rearranged, decomposed, combined, or deleted. Further, steps, measures, and schemes in various operations, methods, and processes in the prior art that are the same as those disclosed in the present invention may also be alternated, modified, rearranged, decomposed, combined, or deleted.

In the description of the present invention, it should be understood that the orientation or locational relationships indicated by terms such as "center", "upper", "lower", "front", "rear", "left", "right", "vertical", "horizontal", "top", "bottom", "inner", "outer", etc., are based on the orientation or locational relationships shown in the drawings. They are used only for convenience in describing the present invention and simplifying the description, and do not indicate or imply that the referred apparatus or element must have a specific orientation or be constructed and operated in a specific orientation. Therefore, they should not be construed as limiting the present invention.

If the terms "first", "second" appear, they are used for descriptive purposes only and should not be construed as indicating or implying relative importance or implicitly indicating the number of the indicated technical features. Thus, features defined by "first" or "second" may explicitly or implicitly include one or more such features. In the description of the present invention, unless otherwise specified, "a plurality of" means two or more.

In the description of the present invention, it should be noted that unless otherwise explicitly specified and defined, the terms "mount", "connect", and "connection" should be understood broadly. For example, a connection may be a fixed connection, a detachable connection, or an integral connection; it may be a direct connection, an indirect connection through an intermediate medium, or an internal communication between two elements. For those of ordinary skill in the art, the specific meanings of the above-mentioned terms in the present invention can be understood based on specific circumstances.

In the description of this specification, specific features, structures, materials, or characteristics may be combined in any suitable manner in one or more embodiments or examples.

Finally, it should be noted that the aforementioned embodiments are only used to illustrate the technical solutions of the present invention, and not to limit them. Although the present invention has been described in detail with reference to the foregoing embodiments, those of ordinary skill in the art should understand that they can still modify the technical solutions described in the foregoing embodiments, or perform equivalent substitutions for some or all of the technical features thereof. However, these modifications or substitutions do not cause the essence of the corresponding technical solutions to depart from the scope of the technical solutions of the embodiments of the present invention.

### INDUSTRIAL APPLICABILITY

By adopting the above scheme, the cause or situation of overpressure is distinguished more carefully through the pressure change rate, so that different adjustment procedures may be adopted. This can largely avoid the problem of high equipment component wear, and targeted adjustment can be made for expected fluctuating overpressure situations, avoiding the problem of long-time consumption for the equipment to stabilize intra-abdominal pressure after expected fluctuation, enabling the intra-abdominal pressure to stabilize quickly, thereby improving surgical efficiency and reducing equipment wear.

## Claims

1. A control method for an insufflator, **characterized by** comprising the following steps:
acquiring a monitored pressure read by an insufflator;
when the monitored pressure is greater than a first pressure threshold and a time duration for which it is maintained is greater than or equal to a first preset time duration, acquiring a pressure change rate within the first preset time duration;
if the pressure change rate is greater than a first pressure change rate threshold, determining it as an expected fluctuation and initiating a first adjustment procedure; and
if the pressure change rate is less than or equal to the first pressure change rate threshold, determining it as continuous change and initiating a second adjustment procedure.

2. The control method for an insufflator according to claim 1, wherein the acquiring a monitored pressure read by an insufflator comprises:
acquiring sampling pressures and sampling time points corresponding to the sampling pressures; and
taking an average value of sampling pressures at every N consecutive sampling time points as the monitored pressure, where N ≥ 1.

3. The control method for an insufflator according to claim 1, wherein the acquiring a pressure change rate within the first preset time duration comprises:
within the first preset time duration, including M values of the monitored pressures, dividing a difference between adjacent monitored pressures by an interval time between the adjacent monitored pressures to obtain M-1 pressure change rates; and
taking a maximum value among the M-1 pressure change rates as the pressure change rate within the first preset time duration.

4. The control method for an insufflator according to claim 1, wherein the first adjustment procedure comprises:
when the monitored pressure is less than or equal to a second pressure threshold and a time duration for which it is maintained is greater than a second preset time duration, initiating a first pressure reduction procedure; and
when the monitored pressure is greater than the second pressure threshold, initiating a second pressure reduction procedure.

5. The control method for an insufflator according to any one of claims 1 to 4, wherein the first adjustment procedure further comprises:
when the monitored pressure is less than the second pressure threshold and the time duration for which it is maintained is less than the second preset time duration, issuing a first prompt signal;
when the monitored pressure is less than or equal to the second pressure threshold and the time duration for which it is maintained is greater than the second preset time duration, issuing a second prompt signal; and
when the monitored pressure is greater than the second pressure threshold, issuing a third prompt signal.

6. The control method for an insufflator according to claim 4, wherein
the first pressure reduction procedure comprises: opening a first pressure relief valve.

7. The control method for an insufflator according to claim 4, wherein
the second pressure reduction procedure comprises: opening the first pressure relief valve and a second pressure relief valve.

8. The control method for an insufflator according to any one of claims 1 to 7, wherein the second adjustment procedure comprises:
when the monitored pressure is less than or equal to the second pressure threshold, initiating a third pressure reduction procedure and initiating a first counting procedure; and
when the monitored pressure is greater than the second pressure threshold, initiating a fourth pressure reduction procedure and initiating a second counting procedure.

9. The control method for an insufflator according to any one of claims 1 to 8, wherein the second adjustment procedure further comprises:
when the monitored pressure is less than or equal to the second pressure threshold, issuing a fourth prompt signal; and
when the monitored pressure is greater than the second pressure threshold, issuing a fifth prompt signal.

10. The control method for an insufflator according to claim 8, wherein
the third pressure reduction procedure comprises: opening the first pressure relief valve.

11. The control method for an insufflator according to claim 8, wherein
the fourth pressure reduction procedure comprises: opening the first pressure relief valve and the second pressure relief valve.

12. The control method for an insufflator according to claim 8, wherein
the first counting procedure comprises: if number of times the third pressure reduction procedure is initiated exceeds a first preset number of times within a third preset time duration, issuing a first warning signal.

13. The control method for an insufflator according to claim 8, wherein
the second counting procedure comprises: if number of times the fourth pressure reduction procedure is initiated exceeds a second preset number of times within a fourth preset time duration, issuing a second warning signal.

14. A control system for an insufflator, **characterized by** comprising:
a pressure monitoring module, configured to acquire a monitored pressure read by an insufflator and determine whether the monitored pressure is greater than a first pressure threshold and a time duration for which it is maintained is greater than or equal to a first preset time duration;
a pressure change rate calculation module, connected to the pressure monitoring module and configured to, when the pressure monitoring module determines that the monitored pressure is greater than the first pressure threshold and the time duration for which it is maintained is greater than or equal to the first preset time duration, acquire a pressure change rate within the first preset time duration;
a determination module, connected to the pressure change rate calculation module and configured to determine whether it is expected fluctuation or continuous change according to the pressure change rate calculated by the pressure change rate calculation module; wherein the determination module is configured to determine it as expected fluctuation when the pressure change rate is greater than a first pressure change rate threshold, and determine it as continuous change when the pressure change rate is less than or equal to the first pressure change rate threshold; and
an adjustment control module, connected to the determination module and configured to be initiated to execute a first adjustment procedure when the determination module determines it as expected fluctuation, and to be initiated to execute a second adjustment procedure when the determination module determines it as continuous change.

15. A control apparatus for an insufflator, **characterized by** comprising a processor, a memory, and a computer program stored in the memory and executable on the processor, wherein the computer program, when executed, implements steps of the control method for an insufflator according to any one of claims 1 to 13.

16. An insufflator, **characterized by** comprising:
the control apparatus for an insufflator according to claim 15;
a pressure sensor, configured to measure an intra-abdominal pressure; and
a first pressure relief valve and a second pressure relief valve, configured to control intra-abdominal pressure relief;
wherein the control apparatus for an insufflator is electrically connected to each of the pressure sensor, the first pressure relief valve, and the second pressure relief valve.
